# EUROPEAN PATENT APPLICATION

(11) **EP 0 982 017 A2**
(43) Date of publication of application: **01.03.2000**
(21) Application number: 99850127.4
(22) Date of filing: 20.08.1999
(51) Int. Cl.: A61F 13/15

(54) **Attachment flaps for sanitary napkin providing a threedimensional form**

(30) Priority: 21.08.1998 SE 9802795
(71) Applicant: SCA Hygiene Products AB, 405 03 Göteborg (SE)
(72) Inventor: Samuelsson, Ann, 437 32 Lindome (SE); Drevik, Solgun, 435 35 Mölnlycke (SE); Österdahl, Eje, 425 58 Västra Frölunda (SE)
(74) Representative: Romare, Laila Anette

(57) **Abstract**

The present invention concerns a sanitary napkin with a front portion (4), a rear portion (5) and an intermediate middle portion (6), comprising an absorbent body (1) enclosed between an upper liquid-permeable surface layer (2) and a lower liquid-impermeable surface layer (3). According to the invention, attachment flaps (11,12) are attached with longitudinal attachment lines (13, 14) to the underside of the liquid-impermeable surface layer (3) on both sides of the longitudinal line of symmetry (A-A) of the sanitary napkin, which attachment flaps are formed so that the attachment lines are made to approach one another when the attachment flaps are attached to each other or to a pair of underpants (17), whereupon the absorbent body, at least within the area for the attachment lines, assumes the form of a longitudinal ridge in the area between the attachment lines.

## Description

### TECHNICAL FIELD

The present invention concerns a sanitary napkin with a front portion, a rear portion and an intermediate portion lying between them, comprising an absorbent body enclosed between an upper liquid-permeable surface layer and a lower liquid impermeable surface layer.

### BACKGROUND OF THE INVENTION

Sanitary napkins which are formed so that their intermediate portion has a ridge-like shape during use are previously known. The aim of such a design is to reduce the risk of excreted fluid being able to run on the surface of the napkin or along the body of the user instead of being absorbed by the absorbent body of the napkin, due to the absorbent body being pressed against the genitals of the user within the area for fluid excretion.

It is known to give the intermediate portions of sanitary napkins a ridge-like form with the aid of pre-stretched elastic elements, the ridge-like form being already formed when the napkin is removed from its packaging. A disadvantage of elastic elements is that their elasticity has a limited duration, which means that the shape of the formed ridge will vary with time. Furthermore, they put a permanent load on the absorbent body and can thus cause it to be deformed in undesired ways. Moreover, the manufacturing process is made more difficult due to the presence of pre-stretched elastic elements in the sanitary napkin.

Another method of achieving the desired ridge-like form in the intermediate portion of the napkin is to utilise the fact that the distance between the inside of the thighs in the crotch of different women varies relatively little. Measurements of sanitary napkin users have shown that there is a space-limiting, critical area in the groin area between two muscle groups which run from the inside of the pelvic floor down along each thigh. The distance between these muscle groups in the genital area has surprisingly been found to vary to a small degree for various individuals with different body shapes and sizes. Even if the distance between a user's thighs obviously is affected by fat, the distance between the muscle groups in the groin of the user is essentially the same irrespective of whether the user is thin, of normal weight or overweight. The distance between the muscle groups has been found to vary between 2.5 and 4 cm, the distance varying between 3 and 3.5 cm in the great majority of individuals.

Moreover, tests have shown that what determines whether a user experiences discomfort in the form of pressure or chafing against the inside of the thighs is whether or not the sanitary napkin has a width in the critical area which significantly exceeds the distance between the above-mentioned muscle groups. The width of the intermediate portion of the absorbent body of a sanitary napkin which uses the above proportions must be at least 4 cm in order to ensure that during use the side edges of the sanitary napkin in the intermediate portion will be subjected to a compressing force which deforms the absorbent body. The compressing force that the thighs exert against the side edges of the sanitary napkin gives the sanitary napkin a tendency to assume a transverse convex form against the body of the user.

A disadvantage of napkins which have a transverse convex form against the body of the user during use, irrespective of how the convex form was formed, is that if a drop of fluid should after all end up on the outside of the surface layer of the napkin in that part of the napkin that does not lie against the body of the user, gravity will help the drop to run over the edge of the sanitary napkin.

An object of the present invention is to solve the problem of allowing a sanitary napkin which, on removal from its packaging, has a flat shape to be given in a simple way a desired transverse convex form. Another object of the invention is to remove the above-mentioned disadvantage of the convex form.

### SUMMARY OF THE INVENTION

This object is achieved in accordance with the invention by means of a sanitary napkin with a front portion, a rear portion and an intermediate middle portion, comprising an absorbent body enclosed between an upper liquid-permeable surface layer and a lower liquid-impermeable surface layer, which is characterised in that attachment flaps are attached with longitudinal attachment lines to the underside of the liquid-impermeable surface layer on both sides of the longitudinal line of symmetry of the sanitary napkin, which attachment flaps are formed so that the attachment lines are made to approach one another when the attachment flaps are attached to each other or to a pair of underpants, whereupon the absorbent body, at least within the area for the attachment lines, assumes the form of a longitudinal ridge in the area between the attachment lines.

In a preferred embodiment, the attachment lines of the attachment flaps extend along the side edges of the napkin at a distance from each respective side edge. Due to the fact that the ridge which is formed when the flaps are attached in this embodiment does not reach as far as the edges of the sanitary napkin, the risk of fluid running over the edge is considerably reduced during use of such a napkin.

In a first variation of this embodiment, the attachment flaps are arranged to be attached to each other by means of cooperating attachment members and have such dimensions that the attachment lines must be brought towards each other in order for the attachment members to be able to cooperate. In an advantageous embodiment, the attachment flaps extend from the attachment line over the underside of the liquid-impermeable surface layer in a direction towards that side edge of the napkin which is opposite the edge along which the attachment line of the attachment flap in question extends. The attachment flaps are preferably made of a pliable material and are shaped so as to be interconnectable with one another, for example by the end portion of one of the attachment flaps being shaped like an arrowhead and the end portion of the other attachment flap comprising a slit, which is shorter than the greatest width of the arrowhead-shaped end portion. In a variation of this embodiment, the attachment flaps extend beyond their point of connection with one another and comprise in their ends attachment members to enable them to be folded round the edge of a pair of underpants and attached to the underside of the underpants.

In a second variation of the preferred embodiment, the first attachment flap has a U-shaped notch in its attachment end and the second attachment flap has an attachment end which is constituted of a central protruding part with a length and breadth which conform to the notch in the first attachment flap, and the attachment flaps comprise in their ends attachment members arranged to be attached to the underside of a pair of underpants and are dimensioned so that the attachment lines must be brought towards each other in order to enable each attachment flap to be folded round the side edge of a pair of underpants and attached to its underside.

The liquid-permeable surface layer can extend round the side edges of the sanitary napkin and in under the liquid-impermeable surface layer until level with the attachment lines of the attachment flaps and the attachment flaps can constitute extensions of the liquid-permeable surface layer in the intermediate portion of the sanitary napkin. Furthermore, folding lines extend along the side edges of the absorbent body at the same distance from each respective side edge as the attachment lines of the attachment flaps, at least in that part of the sanitary napkin in which the attachment lines of the attachment flaps extend. The distance from the attachment lines to the nearest side edge of the absorbent body is 7-14 mm, preferably 10 mm.

### DESCRIPTION OF THE FIGURES

The invention will now be described with reference to the attached figures, in which
- Fig. 1: shows a schematic view from below of a sanitary napkin according to a first embodiment of the invention, in which the attachment flaps are not connected to each other,
- Figs. 2A and 2B: show a cross-sectional view of the sanitary napkin in Fig. 1 with the attachment flaps attached to each other and fixed to the underside and upper side, respectively, of a pair of underpants,
- Fig. 3: shows a similar view to Fig. 1 of a sanitary napkin according to a variation of the first embodiment of the invention,
- Fig. 4: shows a cross-sectional view of a sanitary napkin according to a variation of the sanitary napkin shown in Fig. 3, in which the attachment flaps are not connected to each other,
- Fig. 5: shows a similar view to Fig. 4 but with the attachment flaps connected to each other and attached to the underside of a pair of underpants,
- Fig. 6: shows a similar view to Fig. 1 of a sanitary napkin according to a second embodiment, and
- Fig. 7: shows a variation of the sanitary napkin shown in Fig. 1.

### DESCRIPTION OF EMBODIMENTS

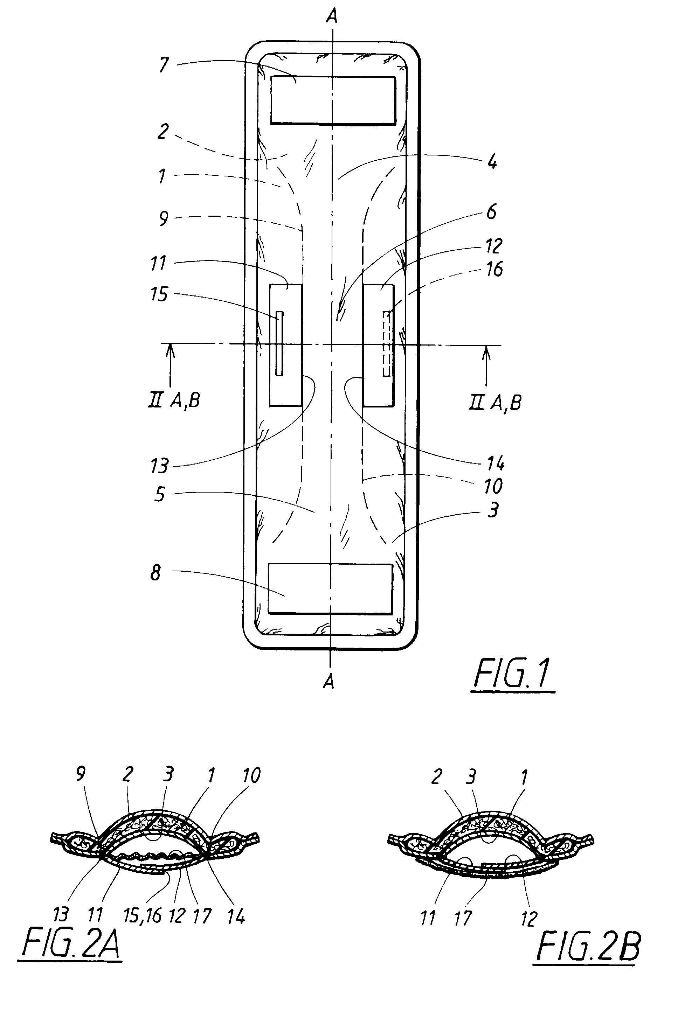

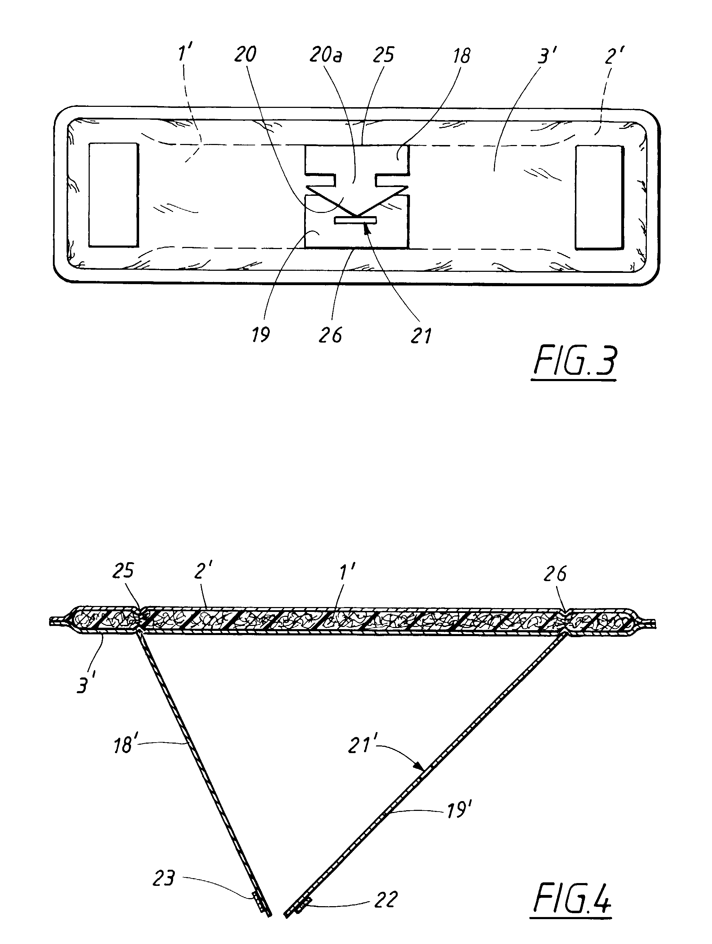

The sanitary napkin shown in Figs. 1 and 2 comprises an absorbent body 1 enclosed between a liquid-permeable surface layer 2 and a liquid-impermeable surface layer 3, at least the liquid-impermeable surface layer being attached to the absorbent body. During use of the sanitary napkin, the liquid-permeable surface layer 2 faces towards the body of the user. Further, the sanitary napkin has a front portion 4, a rear portion 5 and an intermediate middle portion 6. In order to attach the sanitary napkin to the inside of a pair of underpants or briefs, adhesive coatings 7, 8 are arranged on the liquid-impermeable surface layer 3 in the front- and rear portions of the sanitary napkin. The adhesive coatings are covered with a release film or the like, which film is removed before the napkin is attached in a pair of underpants. In addition, folding lines 9, 10 extend along the side edges of the napkin from the intermediate portion and into the front and rear portions.

The sanitary napkin also comprises essentially rectangular attachment flaps 11, 12, which extend in the transverse direction of the napkin in the intermediate portion 6 on the underside of the napkin. The attachment flaps can, of course, have other forms than rectangular, for example, semi-circular, triangular, etc. The attachment flaps 11, 12 are attached to the surface layer 3 along attachment lines 13, 14, which are situated on both sides of the line of symmetry A-A of the napkin and at a distance from each respective side edge of the napkin. In the shown embodiment, the attachment lines 13, 14 are placed at the same distance from the side edges as the folding lines 9, 10, which is a preferred arrangement. Moreover, the attachment flaps 11, 12 are arranged to be attached to each other by means of cooperating attachment members 15, 16.

Furthermore, the attachment flaps 11, 12 are dimensioned in such a way that the attachment lines 13, 14 must be brought towards each other from the position shown in Fig. 1 in order to enable the cooperating attachment members 15, 16 of the attachment flaps to be utilised. This will result in the absorbent body 1 assuming an upwardly convex form seen in the transverse direction, so that a longitudinal ridge is formed within the area between the attachment lines 13, 14. Figs 2A and 2B show a cross-sectional view of the sanitary napkin according to Fig. 1 when adapted for use. In Fig. 2A the attachment flaps 11, 12 are folded round the side edges of a pair of underpants 17 and attached to each other. In this position, the adhesive coatings 7, 8 in the front and rear portions 4, 5 are also attached to the inside of the underpants 17. As can be seen in Fig. 2A, the fact that the attachment lines 13, 14 for the attachment flaps are situated at a distance from the side edges of the napkin means that only the area of the napkin that lies transversely between the attachment lines is affected by the compression force which arises through the attachment lines being brought towards each other. The presence of the folding lines allows the deformation of the absorbent body to be controlled so that the longitudinal ridge which is formed on attachment is certain only to appear between the folding lines even in the front and rear portions. By this means, the side edge portions of the sanitary napkin lying outside the folding lines will retain their essentially horizontal extension when the napkin is attached in a pair of underpants.

When a pair of underpants 17 with the described sanitary napkin applied in it is put on, the edges of the underpants will affect the side edges of the sanitary napkin with an upward force at those places where the edge of the underpants extends under the sanitary napkin out beyond its side edges. If the sanitary napkin has been applied with the flaps attached to one another and placed on the underside of the underpants 17 (Fig. 2A), the edges of the underpants will not extend under the sanitary napkin out beyond its side edges within the area of the attachment flaps but in front of and behind this area. If the sanitary napkin has been applied with the flaps attached to one another and placed on the upper side of the underpants (Fig. 2B), the edges of the underpants will extend under the sanitary napkin out beyond its edges within the area of the attachment flaps. In both cases, the edges of the underpants will affect the side portions of the sanitary napkin lying outside the folding lines with an upwardly bending force.

The attachment flaps 11, 12 can be joined to one another to form a longitudinal ridge on the sanitary napkin before the sanitary napkin is attached in a pair of underpants 17, as is shown in Fig. 2B.

The sanitary napkin can be so dimensioned that the compressive force from the thighs of a user is used to further compress the napkin laterally during use, or it can be dimensioned so that essentially all deformation of the absorbent body occurs during attachment of the sanitary napkin in the underpants. In both cases, the sloping side surface of the longitudinal ridge, which promotes flowing off of any drop of liquid that may appear, becomes a horizontal or upwardly-directed border-like edge portion, which prevents continued lateral liquid flow.

The absorbent body 1 comprises an absorbent material, for example, cellulose fluff with or without the addition of so-called superabsorbent material. Other absorbent materials which are used in absorbent bodies for sanitary napkins can also be used in the absorbent body 1. In the shown embodiment, the absorbent body consists of a single layer of cellulose fluff but the invention shall also include multi-layered absorbent bodies.

The liquid-permeable surface layer 2 is suitably composed of a hydrophobic, hydrophobicised or hydrophilic nonwoven. Other materials which are used as liquid-permeable surface layers for absorbent articles, for example, perforated plastic films and laminates of the mentioned materials, can also be used.

The liquid-impermeable surface layer 3 consists, for example, of a plastic layer or a laminate of nonwoven and plastic, but other materials which are used for liquid-impermeable surface layers, for example microporous plastic films, can naturally also be used.

The attachment flaps 11, 12 can be manufactured of any pliable material, for example, a nonwoven or a plastic film. However, the material must not be so extensible that the resilient force from the deformed absorbent body leads to the attachment flaps, after they have been attached to one another, being stretched so much that the ridge is considerably diminished. The cooperating attachment members 15, 16 can consist of pressure-sensitive glue, in which case only one attachment member 15 is necessary. The attachment members can also be of a mechanical type, for example, fasteners consisting of a hook member cooperating with a loop member. If the attachment flaps are manufactured of nonwoven and if the hook member 15 is of a type that can be directly attached to textile materials, the loop member 16 can be constituted of the flap 12 itself. Such hook members can also be used instead of the adhesive coatings 7, 8. Other mechanical attachment members, such as press studs or the like can naturally also be used to attach the flaps 11, 12 to one another. The attachment members can of course be formed so that the height of the ridge which is formed when the flaps are attached to one another is adjustable, for example, by the attachment member on one of the attachment flaps being constituted of pressure-sensitive glue which can be attached anywhere on the other attachment flap, or by arranging a row of press studs or the like on one of the flaps.

Of course, it is possible to also provide the attachment flap 12 with attachment members on that side which, when the sanitary napkin is applied, faces towards the underside of the absorbent body in order to allow the attachment flaps, after they have been attached to one another, to also be able to be attached to the underside of a pair of underpants (Fig. 2A) or to the underside of the sanitary napkin (Fig. 2B). With this design, the attachment member 15 on the flap 11 is preferably constituted of an attachment member which is attachable to the second attachment flap as well as to the underside of the underpants. The adjustability of the height of the ridge is thus increased from no ridge at all, when the flaps are attached to the underside of the underpants without the attachment lines being pulled towards one another, to maximum height, when the flaps are arranged completely overlapping with one another.

The folding lines 9, 10 are achieved by line compression of the absorbent body 1, Other ways of achieving folding lines, for example removing material from the absorbent body, can, of course, also be used. In the described embodiment of the sanitary napkin, the distance of the folding lines from the nearest side edge of the absorbent body is 7-14 mm, preferably 10 mm, so that the side edge portions of the sanitary napkin which are delimited by the folding lines are compressed to ca 5 mm during use. If the absorbent body were to be composed in another way than is described above, for example, by the absorbent body comprising a stiffening element between the folding lines, the absorbent material in the side edge portions can be softer than in the described embodiment, which implies that the side edge portions can have a greater width than 14 mm without risk of their obtaining a greater width than 5 mm in a compressed condition during use.

Fig. 3 shows a variation of the first embodiment of a sanitary napkin in accordance with the invention. The absorbent body, surface layers, folding lines and adhesive coatings are similar to those in the embodiment described with reference to Figs 1 and 2. All that distinguishes the variations from one another is the form of the attachment flaps. Elements in the variation shown in Fig. 3 which are similar to corresponding elements in the variation shown in Figs. 1 and 2 have been given the same reference numerals as in Figs. 1 and 2 with the addition of a prime.

The attachment flaps 18, 19 in the sanitary napkin shown in Fig. 3 are arranged with such a shape as to be interconnectable with one another and extend from their attachment end transversely a distance in over the underside of the sanitary napkin. As can be seen in the Figure, the flap 18 has an arrowhead-shaped end 20 with a shaft portion 20a and the flap 19 has in its end portion a slit 21 with a length corresponding to the width of the arrowshaft portion 20a of the flap 18. The attachment flaps are of pliable material so that the arrowhead-shaped end portion of the flap 18 can be folded and threaded through the slot 21 and then folded out to lock the flaps to one another. In the same way as for the embodiment shown in Figs. 1, 2A and 2B, the flaps can be attached to one another before the sanitary napkin is attached to a pair of underpants.

The end portion of the flap 18 can, of course, have other forms than that of an arrowhead, for example it can have a rectangular form to be able to lockably cooperate with the slit 21 in the flap 19. What is most important is that the end portion be delimited from the rest of the flap 18 by a part corresponding to the shaft part 20a, which has a lesser width than the rest of the flap.

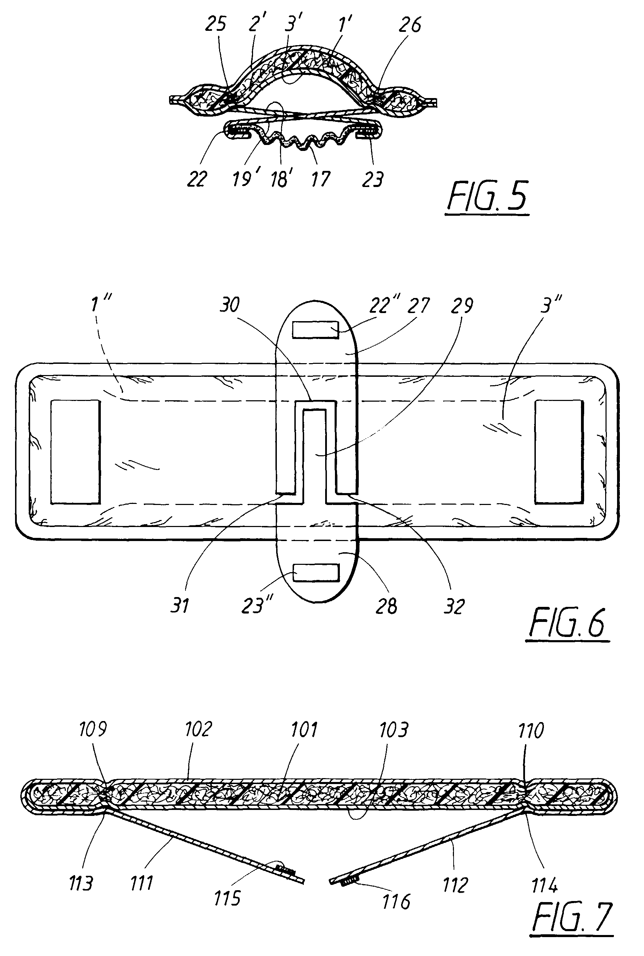

Fig. 4 shows a cross-sectional view of a sanitary napkin conforming to the sanitary napkin shown in Fig. 3 but with attachment flaps 18', 19' constituting variations of the attachment flaps 18, 19 shown in Fig. 3. The flaps 18', 19' differ from the flaps 18, 19 in that they are longer and are intended to be folded round the edge of a pair of underpants and attached to the underside of the underpants. Therefore, the shape-reliant interconnection between the flaps 18', 19' is not arranged in their end portions but instead the end portions comprise attachment members 22, 23 to be attached to the underside of a pair of underpants. In order to apply the sanitary napkin shown in Fig. 4, the flap 18', which comprises a not shown part with lesser width than the rest of the flap, is threaded through the slit 21' in the flap 19'. When the part with lesser width in the flap 18' reaches the slit 21', the flaps are locked to one another and the parts of the flaps lying outside the slit 21' can then be folded over the edge of a pair of underpants 17 and attached to the underside of them, as is shown in Fig. 5.

Fig. 6 shows a sanitary napkin according to a second embodiment. The elements in the sanitary napkin shown in Fig. 6 which are similar to corresponding elements in the sanitary napkin shown in Figs. 1 and 2 have been given the same reference numerals but with the addition of a double prime. All that distinguishes these sanitary napkins from one another is the form of the attachment flaps. In the embodiment shown in Fig. 6, the attachment flap 27 has a U-shaped attachment end, while the attachment flap 28 has an attachment end consisting of a projecting part 29, which fits between the legs of the U-shaped attachment end of the attachment flap 27. The attachment flap 28 is attached to the surface layer 3" along the attachment line 30 and the attachment flap 27 is attached to the surface layer 3" along the attachment lines 31, 32. As in the embodiment shown in Figs. 4 and 5, the attachment flaps 27, 28 extend from their attachment ends transversely over the underside of the sanitary napkin and comprise in their outer ends attachment members 22", 23" in order to attach the flaps to the underside of a pair of underpants after the outer ends have been folded round the side edge of the underpants which is situated on the opposite side of the longitudinal line of symmetry of the napkin to the attachment line of the attachment flap in question. Also in this case, the attachment flaps are so dimensioned that the attachment lines 30 and 31, 32 must be pulled towards one another for the outer ends of the attachment flaps to be able to be folded over the side edge of the underpants. If it is desirable to give the formed ridge a great height, the attachment members should be formed in such a way that they are attachable to the attachment flaps as well as being attachable to the underpants.

Fig. 7 shows a cross-sectional view of a sanitary napkin in accordance with a variation of the embodiment shown in Figs. 1 and 2 in which the attachment flaps 111, 112 constitute an integral part of the liquid-permeable surface layer 102, which extends round the side edges of the absorbent body in under the absorbent body until level with the folding lines 109, 110. The liquid-impermeable surface layer 103 extends in this case round the side edges of the sanitary napkin. In all other aspects, the sanitary napkin in Fig. 7 functions in the same way as the sanitary napkin according to Figs. 1 and 2. The sanitary napkin according to Figs. 3-5 can also be provided in a corresponding way with attachment flaps made in one piece with the liquid-permeable surface layer.

The described embodiments can of course be modified within the frame of the invention, especially with regard to the form of the absorbent body and the extension of the folding lines. For example, the attachment flaps need not be situated in the intermediate portion of the sanitary napkin but can be displaced towards the front or rear portion. The invention shall therefore be limited only by the content of the attached claims.

## Claims

1. Sanitary napkin with a front portion (4), a rear portion (5) and an intermediate middle portion (6), comprising an absorbent body (1; 1'; 1"; 101) enclosed between an upper liquid-permeable surface layer (2; 2'; 102) and a lower liquid-impermeable surface layer (3; 3'; 3"; 103), **characterised** in that attachment flaps (11,12;18, 19; 27,28; 111,112) are attached with longitudinal attachment lines (13, 14; 25, 26; 30, 31, 32) to the underside of the liquid-impermeable surface layer (3; 3'; 3"; 103) on both sides of the longitudinal line of symmetry (A-A) of the sanitary napkin, which attachment flaps are formed so that the attachment lines are made to approach one another when the attachment flaps are attached to each other or to a pair of underpants (17), whereupon the absorbent body, at least within the area for the attachment lines, assumes the form of a longitudinal ridge in the area between the attachment lines.

2. Sanitary napkin in accordance with claim 1, **characterised** in that the attachment lines (13, 14; 25, 26; 30, 31, 32) of the attachment flaps (11, 12; 18, 19; 27, 28; 111, 112) extend along the side edges of the sanitary napkin at a distance from each respective side edge.

3. Sanitary napkin in accordance with claim 1 or 2, **characterised** in that the attachment flaps (11, 12; 18, 19; 111, 112) are arranged to be attached to one another by means of cooperating attachment members (15, 16; 20, 21; 115, 116) and have such dimensions that the attachment lines (13, 14; 25, 26; 113, 114) must be brought towards one another in order for the attachment members to be able to cooperate.

4. Sanitary napkin in accordance with claim 1, 2 or 3, **characterised** in that the attachment flaps (18, 19; 27, 28) extend from the attachment line (25, 26; 30, 31, 32) over the underside of the liquid-impermeable surface layer in a direction towards that side edge of the napkin which is opposite the edge along which the attachment line of the attachment flap in question extends.

5. Sanitary napkin in accordance with claim 4, **characterised** in that the attachment flaps (18, 19) are made of a pliable material and are shaped so as to be interconnectable with one another.

6. Sanitary napkin in accordance with claim 5, **characterised** in that the end portion (20) of one of the attachment flaps (18) is shaped like an arrowhead and the end portion of the other attachment flap (19) comprises a slot (21) which is shorter than the greatest width of the arrowhead-shaped end portion.

7. Sanitary napkin in accordance with claim 5, **characterised** in that the attachment flaps (18', 19') extend beyond their point of connection with one another and comprise in their ends attachment members to enable them to be folded round the edge of a pair of underpants and attached to the underside of the underpants.

8. Sanitary napkin in accordance with claim 4, **characterised** in that the first attachment flap (27) has a U-shaped notch in its attachment end, and in that the second attachment flap (28) has an attachment end which is constituted of a central protruding part (29) with a length and breadth which conform to the notch in the first attachment flap, and in that the attachment flaps comprise in their ends attachment members (22", 23") arranged to be attached to the underside of a pair of underpants and are dimensioned so that the attachment lines (30, 31, 32) must be brought towards each other in order to enable each attachment flap to be folded round the side edge of a pair of underpants and attached to its underside.

9. Sanitary napkin in accordance with any one of claims 1-7, **characterised** in that the liquid-permeable surface layer (102) extends round the side edges of the sanitary napkin and in under the liquid-impermeable surface layer (103) until level with the attachment lines (113, 114) of the attachment flaps (111, 112), and in that the attachment flaps constitute extensions of the liquid-permeable surface layer in the intermediate portion of the sanitary napkin.

10. Sanitary napkin in accordance with any one of the preceding claims, **characterised** in that at least in the part (6) of the sanitary napkin in which the attachment lines (13, 14) of the attachment flaps (11, 12) extend, folding lines (9, 10) extend along the side edges of the absorbent body (1) at the same distance from each respective side edge as the attachment lines (13, 14) of the attachment flaps.

11. Sanitary napkin in accordance with any one of the preceding claims, **characterised** in that the distance from the attachment lines to the nearest side edge of the absorbent body is 7-14 mm, preferably 10 mm.
